# EUROPEAN PATENT APPLICATION

(11) **EP 2 367 005 A1**
(43) Date of publication of application: **21.09.2011**
(21) Application number: 10178983.2
(22) Date of filing: 26.10.2004
(51) Int. Cl.: G01N 33/574

(54) **Method for measuring values indicating cancer**

(30) Priority: 30.10.2003 IT PD20030264
(62) Divisional of application: 04791917.0
(71) Applicant: Xeptagen SPA, 30175 Marghera (VE) (IT)
(72) Inventor: Beneduce, Luca, 30175, MARGHERA (VE) (IT); Marino, Maria, 30175, MARGHERA (VE) (IT); Pontisso, Patrizia, 30175, MARGHERA (VE) (IT); Fassina, Giorgio, 30175, MARGHERA (VE) (IT)
(74) Representative: Vinci, Marcello

(57) **Abstract**

This invention relates to a method for diagnosis of cancer based on the qualitative and quantitative detection of specific circulating immune complexes constituted by tumor associated antigen/ specific autoantibody.

## Description

Cancer is the second major cause of mortality after cardiovascular diseases, with an increasing rate in overall the world.

Tumors have different distribution in the world, but in the industrialized countries distribution and incidence of different types of cancer have similar values. Among men, the incidence rate of prostate and lung cancer is increasing, whereas among woman breast and lung cancer is rising. Both in men and women, other tumors as pancreatic and gastrointestinal tract cancer, are continuously increasing, with an incidence rate for pancreatic cancer which is doubled in the last 50 years.

As a consequence, research in the field of tumor markers in order to find specific molecules associated with cancer and useful in the identification of a neoplasm related to a specific organ, is largely increasing.

When subjected to neoplastic transformation, tumor cells may markedly secrete elevated levels of transformation-related proteins. Such proteins are either formed by or found on the surface of tumor cells, in this case their presence may be detected by histological methods, or, can be found in biological fluids as serum or plasma.

This invention provides a diagnostic method of determining a tumor marker specific for a particular type of cancer, being able to relate blood concentrations of marker with disease staging and treatment monitoring.

At present, no tumor marker satisfy medical requirements in terms of specificity and sensitivity. Instead, it has been found that markers are not specific for a tumor but are also present in healthy subjects. Thus, no test is highly confident because of false positives stemming from release of tumor markers from normal cells and false negatives resulting from the lack of universal tumor markers.

This invention also provides identification of a tumor marker undetectable in healthy subjects.

For this reasons, it would be desirable to provide improved tumor marker assays capable of performing correlation between the presence of the tumor marker and patient's disease state for different type of tumors.

*It has been found that in the serum of patients suffering from cancer, an over-production of tumor antigens as free form and complexed with immunoglobulins (especially immunoglobulins from M class), takes place.*

We have developed assays to assess the presence of circulating immune-complex composed by the association of biomarker/specific auto-antibody (hereinafter referred to as specific CIC), useful for tumor diagnosis. Compared to biomarker detection as free antigen, such method allows increasing diagnostic sensitivity, being capable to discriminate from healthy subjects and those affected by disease.

As a rule, self-reactive auto-antibodies are produced by the immune system in response to antigen over-expression occurring in tumors and not in the healthy subjects (Sahin U. et al. Proc. Natl. Acad. Sci. USA, 1995, 92, 11810-13).

Therefore, there are some evidences that the detection of free antigen doesn't have diagnostic relevance, but when the antigen is coupled to the autoantibody to form the immune-complex (IC), becomes more specific being capable to discriminate healthy condition from disease with a specificity of 100%.

It is believed that the formation of these complexes may have two effects which are related to the detection of tumor associated antigen in the bloodstream. First, when the antigen is involved in such complexes, the antigen may be refractory to detection by normal immunoassays. This situation would lead to false negative results as observed in the prior art. Second, because low levels of the antigen released by normal cells will usually not form persistent immune complexes, the free antigen may be detected, giving false positive results. Both these sources of error may be obviated by detecting tumor associated antigen only as circulating immune complexes (CIC) as accomplished by the method of the present invention.

The method herein described allows overcoming these problems and detecting the presence of tumor marker by using specific reagents.

A great number of cancer diseases where tumor associated antigens are known, has been studied and it has been demonstrated that detection of immune-complexes composed by specific tumor antigen/specific autoantibody, has a diagnostic relevance for a given tumor. **Table 1** shows some of neoplastic diseases where the new method may be applied.

Only few biomarkers shown in table 1 are used in the clinical practice with diagnostic relevance. The alpha-fetoprotein (AFP) which is used as biomarker of first choice (see table 2) for diagnosis of hepatocellular carcinoma, has low sensitivity and specificity since its value may be elevated in patients with chronic liver disease.

**Table 2** shows biomarkers used in the clinical practice.

Therefore, a first aspect of this invention relates to a method for cancer detection based on the determination of specific CIC in biological samples from patients, by contacting and incubating a sample obtained from subject with specific reagents (SR) for the tumor associated antigen, determining the presence or absence of the specific RS-CIC, whereby the presence of said complex is indicative of disease.

As shown in the following examples, the object herein disclosed has been widely validated on a tumor disease of liver: the hepatocellular carcinoma.

Hepatocellular carcinoma (HCC) is one of the most widespread diseases in the world and its prognosis is particularly severe since only about 3% of patients survive after five years from diagnosis.

A problem related to this disease is the difficulty to have an early diagnosis in order to carry out a timely intervention.

Currently, alpha-fetoprotein (AFP) is the only serologic parameter used in diagnostic screening of HCC, even if its specificity and sensitivity are not satisfactory. In fact, the values of AFP are elevated in the 50-80% of patients with HCC, but can be slightly elevated also for patients with hepatic cirrhosis without tumor, or can be normal also in the presence of isolated tumoral nodules that can be evidenced by ultrasound examinations.

For this reason it is recommended to screen patients with cirrhosis by liver ultrasonography every 6 months.

**Table 1**

| **Tumor markers** | **Tumor** | **References** |
|---|---|---|
| Ki-67 | Astrocytoma | Br J Cancer 89(1):128-34, 2003 |
| Fibronectin | Bladder cancer | ClinChemLab Med 41(8):1069-74, 2003 |
| Hepatoma up regulated protein (HURP) | Bladder cancer | Anticancer Res 23(3B):2729-33, 2003 |
| Mucin 7 (MUC7) | Bladder cancer | Urology 62(1):182-86, 2003 |
| NMP22 | Bladder cancer | J Chin Med Assoc 66(5):294-98, 2003 |
| NMP22 | Bladder cancer | Anticancer Res 23(2A):805-12, 2003 |
| Prostate stem cell antigen (PSCA) | Bladder cancer | J Urol 169(6).2094-100, 2003 |
| Telomerase | Bladder cancer | Urology 62(2).362-67, 2003 |
| Tissue polypeptide antigen (TPA) | Bladder cancer | Urology 62(2):243-48, 2003 |
| CA 15-3, CA 27.29, AFP, CEA | Breast cancer | Biomed Sci Instrum 39:408-14, 2003 |
| CA 15-3, Ceruloplasmin, TPA | Breast cancer | East Afr Med J 77(6):291-94, 2000 |
| CEA, Cytokeratin 19 (CK19), Maspin | Breast cancer | Anticancer Res 23(2C):1883-90, 2003 |
| c-Met | Breast cancer | Breasr Cancer Res 5(3):71-76, 2003 |
| Cytochrome P450 3A4 | Breast cancer | Biomed Sci Instrum 39:24-29, 2003 |
| Epithelial glycoprotein 2 (EGP2), Cytokeratin 19 (CK19) | Breast cancer | Int J Cancer 106(4):611-18, 2003 |
| ErbB-2 | Breast cancer | Cancer Lett 193(2):139-48, 2003 |
| Her2/Neu | Breast cancer | Anticancer Res23(2A):999-1006, 2003 |
| MMP-9 | Breast cancer | Int J Cancer 106(5):745-51, 2003 |
| Human kallikrein 5 (hK5) | Breast, ovarian cancer | Cancer Res 63(14):3958-65, 2003 |
| NMP179 | Cervical cancer | Anticancer Res 23(2A):805-12, 2003 |
| CEA, Cytokeratin 19 (CK19), Cytokeratin 20 (CK20) | Colorectal cancer | J Cancer Res Clin Oncol 129(3):192-98, 2003 |
| Cytokeratin | Colorectal cancer | Colorectal Dis 5(2):164-68, 2003 |
| Cytokeratin 20 (CK20), CEA, Guanylyl cyclase C (GCC) | Colorectal cancer | Eur J Cancer 39(9):1234-41, 2003 |
| Her2/Neu | Colorectal cancer | Int J Cancer 105(6):796-802, 2003 |
| MUC6, MUC5AC | Colorectal cancer | Glycocong J 18(11-12):907-14, 2001 |
| RelA, NF-kb | Colorectal cancer | Oncology 65(1):37-45, 2003 |
| Bcl-6, CD10 | Diffuse largeb-cell lymphoma | Human Pathol 34(6):610-16, 2003 |
| CEA | Endometrial carcinoma | Anticancer Res 23(2A):1103-06, 2003 |
| Cystein-rich fibroblast growth factor receptor 1 (CFR-1) | Gastric cancer | Cancer Res 63(9):2052-61, 2003 |
| p27, MIB-1 | Gastric cancer | Pathologica 95(1):22-30, 2003 |
| Chromogranin A (CgA), Neuron specific enolase (NSE), Synaptophysin, Leu-7, beta III-tubulin | Gastrointestinal carcinoma | Cesk Pathol 39(2):47-53, 2003 |
| Cytokeratin, Epithelial membrane antigen (EMA) | Head and neck carcinoma | Laryngoscope 113(5):892-96, 2003 |
| Hyaluronidase (HYAL1) | Head and neck carcinoma | Int J Cancer 106(3):438-45, 2003 |
| Latent membrane protein 1 (LMP-1) | Head and neck carcinoma | Cancer 97(8):1909-13, 2003 |
| Alpha-fetoprotein (AFP), Des-gamma-carboxy prothrombin (DCP) | Hepatocellular carcinoma | Eur J Gastroenterol Hepatol 15(6):641-48 |
| Cellular retinol binding protein 1 (CRBP1) | Hepatocellular carcinoma | Hepatology 38(2):470-80, 2003 |
| Glypcan-3 | Hepatocellular carcinoma | Gastroenterology 125(1):89-97, 2003 |
| Telomerase | Hepatocellular carcinoma | Oncology 64(4):430-34, 2003 |
| Preferentially expressed antigen of melanoma (PRAME) | Leukemias,Multiple myeloma | Leuk Lymphoma 44(3):439-44, 2003 |
| CEA, Chromogranin A, NSE, Vascular endothelial growth factor (VEGF), Stem cell factor (SCF), Hepatocyte growth factor/Scatter factor (HGF/SF) | Lung cancer | Anticancer Res 23(1A):49-62, 2003 |
| Epidermal growth factor receptor (EGFR) | Lung cancer | Int J Clin Oncol 8(2):79-82, 2003 |
| M2-PK, CYFRA 21-1, NSE, SCC | Lung cancer | Anticancer Res 23(2A):899-906, 2003 |
| Neuron specific enolase (NSE), S-100B, Tyrosinase, LDH | Melanoma | Magy Onkol 47(1):89-104, 2003 |
| Cytokeratin 20 (CK20), Prostate stem cell antigen (PSCA) | Miscellaneus cancers | Anticancer Res 23(3B):2711-16, 2003 |
| Medkine (MK) | Gastric cancer | BBRC 306(2):329-32, 2003 |
| CA 125 | Ovarian cancer | Oncology 65(1):1-6, 2003 |
| CASA, CA 125 | Ovanan cancer | Anticancer Res 23(2A):1115-18, 2003 |
| CA 19-9 | Pancreatic carcinoma | Anticancer Res 23(2A):835-40, 2003 |
| Survivin, p53, Bcl-2 | Pancreatic carcinoma | Int J GastrointestCancer32(2-3):73-81, 2002 |
| Secretogranin II-derived peptide EM66 | Pheochromocytoma | J Clin Endocrinol Metab 88:2579-85, 2003 |
| GLUT1, GLUT12 | Prostate cancer | Cancer 97(8):2035-42, 2003 |
| Insulin-like growth factor binding protein 2 (IGBFB2) | Prostate cancer | Virchows Arch 442(4):329-35, 2003 |
| Myogenin, MyoD1 | Rhabdomyosarcoma | J Clin Pathol 56(6):412-16, 2003 |
| Dipeptidyl Peptidase IV (DPP IV/CD 26) | Thyroid cancer (papillary carcinoma) | Neoplasma 50(3):159-64, 2003 |
| Peroxisome proliferating activated receptor gamma (PPAR gamma) | Thyroid cancer (papillary carcinoma) | Am J Clin Pathol 120(2):175-81, 2003 |

Major limitations of this method are related to the difficult technical detection of tumors with 1 cm of diameter and tumors with the same density as normal liver tissue which are visible only with additional cunning devices not routinely employed. As a consequence, more invasive methods, like biopsy, have to be used.

The availability of a specific marker for HCC that can be detected in biological fluids, might allow an early diagnosis, disease monitoring and screening strategies on at-risk population, by only taking blood samples.

Surprisingly, it has been found that the antigen associated to the HCC is constituted by related variants of the squamous cell carcinoma antigen, over-expressed in hepatic tissues characterized by malignant transformation. Such antigens have been previously identified as proteins iperexpressed in malignant liver tissue, and an immunohistochemical method for detecting of has been previously disclosed (see "Anticorpi policlonali" N0. 1.317.355, Xeptagen).

Therefore, a second aspect of this invention relates to a method for measuring values indicating liver cancer, based on the detection of circulating immune-complexes containing protein variants belonging to serpin family, and immunoglobulins.

In addition to circulating antigens also pre-neoplastic cells are present in many tumor diseases.

A third aspect of this invention relates to a method of detecting the presence of pre-neoplastic cells in subjects suspected of having pre-neoplastic cells which produce cancer marker protein by contacting and incubating a sample obtained from the subject with SR against tumor associated antigen, determining the presence or absence of the specific complex SR-CIC, whereby the presence of such a complex is indicative of the presence of cancer.

A fourth aspect of this invention relates to a method for measuring marker values indicating the HCC based on the detection of tumor antigens involved in the formation of specific circulating immune-complexes in biological fluids of patients, by using poly- and/or monoclonal antibodies in a immune-enzymatic assay.

Examples of standard immunometric methods suitable for detecting CIC in a biological fluid include, but are not limited to, direct, indirect or sandwich ELISA (enzyme linked immunosorbent assay), radio-immunoassay (RIA), and "sandwich" immune radiometric assays (IRMA), or homogeneous immunoassays based on other technologies like 'rapid-near-infrared spectroscopy'.

**Table 2**

| **Tumor marker** | **Cancer** | **Clinical relevance** |
|---|---|---|
| CEA | Lung, Tyroid And Uterine Adenocarcinoma, Colorectal Cancer, Ovarian Adenocarcinoma (I Choice) Stomach Carcinoma (II Choice) Pancreatic Carcinoma (II Choice) Bile Duct Carcinoma (II Choice) Esophagus Carcinoma (II Choice) Breast Carcinoma (II Choice) | Prognostic value, treatment monitoring |
| AFP | Germinal testis tumors Ovarian carcinoma Hepatocellular carcinoma | Differential diagnosis, prognostic value and treatment monitoring |
| TPA | Head and neck squamous cell carcinoma Esophagus Carcinoma Colorectal Cancer (II choice) Bladder carcinoma (I choice) | Prognostic value, treatment monitoring |
| TPS | Breast cancer (II choice) | Prognostic value, treatment monitoring |
| CYFRA21.1 | Spinocellular lung Carcinoma | Prognostic value, treatment monitoring |
| CA125 | Serous ovarian adenocarcinoma (I choice) | Differential diagnosis, prognostic value and treatment monitoring |
| CA19.9 | Colorectal carcinoma (II choice) Bladder carcinoma Bile Duct Carcinoma, Pancreatic carcinoma, Stomach Carcinoma (I choice) | Prognostic value, treatment monitoring |
| CA50 | Kidney carcinoma (II choice) | Prognostic value, treatment monitoring |
| CA72.4 | Stomach Carcinoma (I choice) | Prognostic value, treatment monitoring |
| CA15.3 | Breast carcinoma (I choice) | Prognostic value, treatment monitoring |
| MCA | Kidney carcinoma (II choice) | Prognostic value, treatment monitoring |
| SCC | Esophagus Carcinoma Head and neck squamous cell carcinoma (II choice) Uterine carcinoma (I choice) | Prognostic value, treatment monitoring |
| HCG | Germinal testis tumors Ovanan carcinoma Uterine carcinoma (vescicular mole) (I choice) | Differential diagnosis, prognostic value and treatment monitoring 1 |
| TG | Thyroid cancer (I choice) | Prognostic value, treatment monitoring |
| CT | Thyroid cancer (I choice)) | Differential diagnosis, prognostic value and treatment monitoring |
| PAP total PSA Free PSA | Prostate cancer (I choice) | Differential diagnosis, prognostic value and treatment monitoring |
| NSE | Lung carcinoma (microcytoma) (I choice) Melanoma (II choice) | Differential diagnosis, prognostic value and treatment monitoring |
| FERRITIN | Hepatocellular carcinoma (II choice) | Prognostic value, treatment monitoring |
| S-100 | Melanoma (I choice) | Prognostic value, treatment monitoring |

A typical example for immunometric assay to detect CIC in biological fluids comprises:
1] immobilization of specific reagents for tumor marker on the ELISA plate plastic surface, or on particles that can be used for diagnostic purpose, such reagents may be used as free or molecules conjugated form;
2] contacting the specific coated reagent with patient biological fluid
3] washing out exceeding molecules
4] incubation of the SR-CIC with poly- or monoclonal antibodies against human immunoglobulins. Such antibodies can be conjugated with a system generating signal such as enzymes or chromogenic or fluorescent or radioactive or chemioluminescent compounds or coenzymes or enzyme inhibitors or other systems for detection known from literature;
5] washing out exceeding antibodies;
6] detection of the adsorbed antibody by using suitable reagents.

Examples of specific reagents (SR) against tumor antigen might be poly- or/and monoclonal antibodies or other ligands as lectins which specifically recognize the carbohydrate moieties of the tumor antigen.

Samples useful for determination are body fluids such as serum, plasma, lymph, ascites, pleural effusion, cerebrospinal fluid etc... Such a body fluid can be collected in the usual manner from the desired patient in need of such determination.

All the procedures referred above are well described in the literature and are known by expert in this field.

The invention will be described by the following examples.

### Example 1. Production of SCCA1-specific polyclonal antibodies developed in rabbits.

Recombinant or extractive SCCA1 protein was used to immunize New Zeland White rabbits by intradermal injections. A boost with the same antigen was carried out after four weeks and after 2 months serum samples were taken in order to obtain the serum fraction containing specific antibodies. Such fraction was assayed for the presence of specific anti-SCCA1 antibodies by ELISA technique.

Microtiter plates for ELISA assay (Falcon Cat. No 3912) were coated with 50 µL of 10 µg/mL SCCA1 solution in bicarbonate buffer pH 9.6 for 12 hours at 4°C. Plate were washed with 150mM sodium phosphate buffer, pH 7.2 (PBS) and then each well was filled with 200µL of PBS solution containing 3% of bovine serum albumin (PBS-3%BSA) (BSA, Sigma Cat No A-4503). After incubation for 2 hours, plates were washed three times with PBS containing 0.05% Tween 20 (PBS-T) and filled with anti-SCCA1 rabbit serum solution previously diluted in PBS. Microtiter plates were then incubated for 1 hour at 37°C, washed six times with PBS-T, and 50µL of horseradish peroxidase-labeled goat anti-rabbit immunoglobulins (Sigma, Cat. No A-6154) solution, diluted 1000-fold with PBS-T containing 1% BSA (PBS-T-1%BSA), was added to each well.

After 1 hour of incubation at 37°C, plates were washed again with PBS-T, and then 100µL of 1 mg/mL o-phenylendiamine dihydrochloride (Sigma Cat No P-6912) dissolved in 0.1 M sodium citrate buffer containing 5mM hydrogen peroxide, were added to each well. The color was allowed to develop for 30 min and then the reaction was stopped by adding 25µL of 4.5 M sulfuric acid solution. Absorbance at 492 nm was determined by an ELISA plate reader (BioRad).

Other serpin variants were used in order to produce specific polyclonal antibodies by using the same technique.

### Example 2. Production of SCCA1-specific monoclonal antibodies.

Four weeks old Balb/c mice (Harlan Nossan, Milano, Italia) were immunized with 50 µg of SCCA1 protein of recombinant or extractive origin. In the following two months, animals were boosted every two weeks with the same amount of protein. After each boost anti-SCCA1 antibody titer was monitored by ELISA assay using biotinylated SCCA1, and on the basis of immunoreactivity one mouse was chosen for hybridoma preparation at the end of the immunization procedure. Murine myeloma cells used for hybridoma preparation were NS0 cultured in the following conditions: DMEM (Sigma-Aldrich, Milano, Italia, D-5671) + 10% FCS (Sigma-Aldrich, Milano, Italia, F-7524) + 1% NEAA (Sigma-Aldrich, Milano, Italia, M-7145) + 2 mM Glutamine (Sigma-Aldrich, Milano, Italia, G-7513) + 1% Penicillin/Streptomycin (Sigma-Aldrich, Milano, Italia, P-0871). NS0 cells do not secrete any antibody and are negatively selected with HAT medium.

The mouse with the highest titer was selected and sacrificed in a CO₂ chamber. Spleen was aseptically removed, placed in a tissue culture dish and washed with 10 mL of DMEM containing 2 mM Glutamine, 1% NEAA (DMEM-GM). Thus, the spleen was mechanically broken up and the obtained suspension was placed in a tissue culture dish containing 5 mL of DMEM-GM prewarmed at 37°C. The remaining clumps were broken up with 5 mL pipette, and cells were recovered by centrifugation, washed with the same medium and counted by a Burker chamber. NS0 cells were counted and resuspended in DMEM-GM medium, and added to splenocytes in a 1:5 ratio. The cell mixture was recovered by centrifugation, washed again with DMEM-GM medium, and in order to enhance fusion, 1 mL of DMEM-GM medium containing 44% PEG (Sigma-Aldrich, Milano, Italia, P-7777) was added to the pellet in 1 min. Two milliliters of the same medium were added in the next 3 min, and after pellet resuspension, 7 mL of DMEM-GM + 10% FCS (Sigma-Aldrich, Milano, Italia, F-7524) were added under stirring. Cells were recovered by centrifugation and gently resuspended with 10 mL of DMEM-HAT [DMEM-GM+ 15% FCS + 2% HAT 50x (Sigma-Aldrich, Milano, Italia, H-0262) + ECGS (Sigma-Aldrich, Milano, Italia, n° cat, E-0760) + 1% Penicillin/Streptomycin (Sigma-Aldrich, Milano, Italia, P-0871), then cell mixture was diluted and dispensed in 10 microtiter plates.

After about 15 days clone supernatants were analyzed by ELISA assay in order to evaluate SCCA1-specific antibody production by using biotinylated SCCA1. Positive clones were amplified, secreted monoclonal antibodies were characterized and the isotype determined. Supernatants were purified by the method described in the example 3.

The specific antibody production has been evaluated by the ELISA assay described below.

Microtiter plates for ELISA assay (Falcon Cat. No 3912) were coated with 50 µL of a 40 µg/mL solution of goat anti-mouse immunoglobulins (Sigma-Aldrich, Milano, Italia, n° cat. M-3014) in PBS buffer pH 7.2 for 12 hours at 4°C. Plate were washed with 150 mM PBS, pH 7.2 and then each well was filled with 200µL of PBS solution containing 3% of bovine serum albumin (PBS-3%BSA) (BSA, Sigma Cat No A-4503). After incubation for 2 hours, plates were washed three times with PBS containing 0.05% Tween 20 (PBS-T) and filled with supernatants previously diluted in PBS. Microtiter plates were then incubated for 1 hour at 37°C, washed six times with PBS-T, and 50µL of biotinylated-SCCA1 solution at a concentration of 10 nM were added to each well. After 1 hour incubation at 37°C, plates were washed and a solution of 1 mg/mL of horseradish peroxidase-labeled streptavidin (Sigma-Aldrich, Milano, Italia, n° cat. E-2886) was added to each well.

After 1 hour of incubation at 37°C, plates were washed again with PBS-T, and then 100µL of ABTS (Sigma-Aldrich, Milano, Italia, n° cat 11565) solution were added to each well. The color was allowed to develop for 15 min and then the absorbance at 405 nm was determined by an ELISA plate reader (BioRad).

Other serpin variants were used in order to produce specific monoclonal antibodies by using the same technique.

### Example 3. Purification of polyclonal and monoclonal antibodies by affinity chromatography on SCCA1-immobilized columns.

SCCA1 protein of recombinant or extractive origin (5 mg), was dissolved in 5 mL of 0.1 M sodium bicarbonate butter, pH 9.0, and then added to 1.2 g of NHS-Activated Sepharose 4 Fast Flow (Amersham Pharmacia Biotech, n° cat. 17-0906-01) matrix designed for affinity chromatography suitable for peptide and protein immobilization. The suspension was mixed for 4 hours and coupling extend was monitored taking samples at different time and analyzing them by RP-HPLC. About 85% of the initial protein was attached to the matrix by formation of stable bonds after 4 hours. Derivatized resin was washed with 50 mL of a solution of 1 M TRIS pH 9.0, and then packed in a 100x 6.6 mm I.D. column. For purification procedures, columns were equilibrated with 50 mM TRIS pH 7.0, at a flow rate of 1.0 mL/min monitoring the effluent by UV at 280 nm. One milliliter of crude rabbit serum containing anti-SCCA antibodies was applied to the column and after washing out of unbound proteins, buffer applied was changed to 0.1 M acetic acid pH 4.0. The adsorbed proteins were collected and analyzed by SDS-Polyacrylamide gel electrophoresis. Monoclonal antibodies against SCCA 1 protein were purified in the same manner, loading the column with samples from cell culture supernatants obtained from hybridoma, using the same elution procedures described above.

### Example 4. Detection of specific CIC formed by SCCA1 protein and/or its protein variants and specific autoantibodies, in the serum samples from HCC patients.

Microtiter plates for ELISA assay (Greiner High Binding) were coated with 10 µg/mL of rabbit anti-human SCCA antibody in 100 µL PBS pH 7.2 for 12 hours at 4°C. Plates were washed with PBS containing 0.05% Tween 20 (PBS-T) and then each well was filled with 200µL of PBS solution containing 1% of bovine serum albumin (PBS-1%BSA) (BSA, Sigma Cat No A-4503). After incubation for 2 hours, plates were washed six times with PBS-T, filled with 100 µL of serially diluted samples in PBS-1%BSA and then incubated for 1 hour at 37°C. To quantify the CIC concentration in serum, a calibration curve of purified CIC (SCCA-IgM) with concentration range between 15 and 1000 AU/mL, was performed in parallel. As negative control, PBS-T containing 1% BSA (PBS-T-1%BSA) was used. After 1 hour incubation at room temperature, plates were washed six times with PBS-T, and 100µL of horseradish peroxidase-labeled goat anti-human immunoglobulin (Sigma, Cat. No A-6154) solution, diluted 1000-fold with PBS-T PBS-T-1%BSA, was added to each well.

After 1 hour of incubation at room temperature, plates were washed again with PBS-T, and then 200µL of ABTS (Sigma-Aldrich, Milano, Italia, cat. No. 11565) were added to each well. The color was allowed to develop for 20-30 min and then the absorbance at 405 nm was determined by an ELISA plate reader (BioRad). Sample concentrations, expressed in Arbitrary Units/mL (AU/mL) were determined by interpolation of sample absorbances on the standard curve. Analysis of HCC patient sera confirmed the presence of specific CIC, whereas all control sera from healthy subjects were negative (100% specificity).

Same results were obtained employing the assay previously described detecting CIC formed by protein variants related to SCCA1 belonging to serpin family.

The method described in this invention is useful to diagnose progression disease.

This invention also provides a kit containing reagents useful to perform the assay described above. In preferred form, kit includes a specific reagent for SCCA1 protein. The kit may also contain reagents with high specificity and affinity for serpin protein variant and reagents conjugated with signal amplification system.

## Claims

1. Method for measuring values indicating cancer, **characterized in that** it comprises the measurement of specific circulating immune complexes (CIC) associated to the cancer, present in the patient blood, said cancer being represented by prostate cancer, where specific CIC are formed by marker associated to prostate cancer and specific autoantibody, said marker being represented by a protein belonging to kallikrein family, and wherein said method comprises the use of antibodies against prostate specific antigen (PSA), the entire protein or a part of, and closely related variants.

2. Method for measuring values indicating cancer according to claim 1, **characterized in that** it is an immune-enzymatic method.

3. Method for measuring values indicating cancer according to claim 2, **characterized in that** it is an ELISA immune assay.

4. Method for measuring values indicating cancer according to claim 2, **characterized in that** it is a RIA "radio- immune assay".

5. Method for measuring values indicating cancer according to claim 2, **characterized in that** it is an IRMA "sandwich immune-radiometric assay".

6. Method for measuring values indicating cancer according to claim 2, **characterized in that** it is used in combination with an amplification system able to increase test sensitivity.

7. Method for measuring values indicating cancer according to previous claim, **characterized in that** said amplification system comprises an enzyme amplification technique.

8. Method for measuring values indicating cancer according to claim 1, **characterized in that** said autoantibodies constituting the CIC, are represented by IgM.

9. Method for measuring values indicating cancer according to claim 1, **characterized in that** said autoantibodies constituting the CIC, are represented by IgG.

10. Method for measuring values indicating cancer according to claim 1, **characterized in that** it comprises the use of antibodies for diagnosis of prostate cancer.

11. Method for measuring values indicating cancer according to claims from 1 to 10, **characterized in that** it comprises the use of antibodies wherein said antibodies are for diagnosis of prostate cancer from patient urine.

12. Method for measuring values indicating cancer according to claims from 1 to 11, **characterized in that** it comprises the use of antibodies wherein said antibodies are polyclonal antibodies.

13. Method for measuring values indicating cancer according to claim 12, **characterized in that** it comprises the use of antibodies wherein said antibodies are generated in rabbits or other animal.

14. Method for measuring values indicating cancer according to claim 13 **characterized in that** it comprises the use of antibodies wherein said antibodies are monoclonal antibodies.

15. Method for measuring values indicating cancer according to claim 14, **characterized in that** it comprises the use of antibodies wherein said antibodies are generated in mice or other animal.

16. Method for measuring values indicating cancer according to claims from 12 to 15, **characterized in that** it comprises the use of antibodies wherein said antibodies are used as antibody fragments.
